# EUROPEAN PATENT SPECIFICATION

(11) **EP 3 137 040 B1**
(45) Date of publication and mention of the grant of the patent: **24.08.2022**
(21) Application number: 14726306.5
(22) Date of filing: 30.04.2014
(51) Int. Cl.: A61K 8/24, A61K 8/25, A61K 8/27, A61K 8/362, A61K 8/81, A61K 8/02, A61Q 11/00

(54) **ORAL CARE COMPOSITION CONTAINING SILICA AND ZINC CITRATE**
MUNDPFLEGEZUSAMMENSETZUNG MIT SILICIUMDIOXID UND ZINKCITRAT
COMPOSITION DE SOIN BUCCAL CONTENANT DE LA SILICE ET DU CITRATE DE ZINC

(43) Date of publication of application: 08.03.2017
(73) Proprietor: Colgate-Palmolive Company, New York, NY 10022 (US)
(72) Inventor: PRENCIPE, Michael, Princeton Junction, New Jersey 08550 (US); FISHER, Steven Wade, Middlesex, New Jersey 08846 (US)
(74) Representative: Wibbelmann, Jobst
(86) International application number: PCT/US2014/036023
(87) International publication number: WO 2015/167488

(56) References cited:
- EP-A1- 0 331 415
- WO-A2-02/45678
- WO-A2-2007/076444
- US-A- 4 100 269
- US-A1- 2008 267 891

## Description

### BACKGROUND

Dental plaque is a biofilm that adheres to tooth and other oral surfaces, particularly at the gingival margin, and is implicated in the occurrence of gingivitis, periodontitis, caries and other forms of periodontal disease. Dental plaque is cohesive and highly resistant to removal from teeth and/or oral surfaces. Dental plaque comprises glucans, which are insoluble polysaccharides that provide plaque with its cohesive properties. The bacterial enzyme glucosyltransferase converts dietary sugar into glucans. Plaque mineralizes to form a hard deposit called calculus (or tartar), which becomes a local irritant for the gums, causing gingivitis.

Various antibacterial agents can retard the growth of bacteria and thus reduce the formation of biofilm on oral surfaces.

Zinc and other metal compound/salts have been previously used as antibacterial agents. Without being bound by any theory, free zinc ions are believed to provide antibacterial efficacy by inhibition of glucose metabolism and/or interaction with the bacterial cell wall, reducing bacterial colonization of the oral cavity (as discussed in Cummins D., J Clin Periodontol 1991; 18; 455-461). An insoluble zinc compound, zinc oxide, could also deliver strong antibacterial efficacy during tooth brushing.

Dentinal hypersensitivity is a temporary induced pain sensation produced when hypersensitive teeth are subjected to changes in temperature and/or pressure or to chemical action. Hypersensitivity may occur whenever the dentin of a tooth is exposed by attrition or abrasion, or when the tooth's finer root surface is exposed by periodontal disease. Dentin is a bone-like material in teeth that is usually covered by enamel above the gum line and cementum below the gum line. The enamel or cementum may be removed through decay, injury, disease or other causes, thereby exposing the dentin to external stimuli in the mouth. Dentin generally contains channels, called tubules, that allow material and energy transport between the exterior of the dentin and the interior of the tooth where the nerve is located.

One theory of dentinal hypersensitivity, called the hydrodynamic theory, suggests that exposure of these tubules to external stimuli can cause irritation of the nerve and lead to the discomfort of hypersensitivity. The hydrodynamic theory suggests that hypersensitivity may be treated by making the nerve in the tooth less sensitive to stimuli, or by blocking or occluding the tubules to prevent or limit exposure of the nerve to external stimuli.

Many attempts have been made to control dentinal hypersensitivity. One approach is to reduce the excitability of the nerve in a sensitive tooth. Another approach to control dentinal hypersensitivity, as discussed above with regard to the hydrodynamic theory, is the use of agents to fully or partially occlude tubules. Use of silica particles having an average particle size of no greater than the diameter of a dentin tubule for occluding dentinal tubules and treating dentinal hypersensivity is disclosed in US patent application no. 2009/0092562.

It would be desirable to provide improved oral care compositions which exhibit biofilm reduction efficacy and provide dentinal desensitizing efficacy.

### BRIEF SUMMARY

Provided is an oral care composition comprising: (a) a silica abrasive having an average particle size of 8 microns or less;(b) zinc citrate,(c) a bioadhesive agent comprising a polyvinyl methyl ether/maleic anhydride copolymer, and(d) an anti calculus agent comprising tetrapotassium pyrophosphate and tetrasodium pyrophosphate.

Also provided is an oral care composition for use in reducing or inhibiting biofilm formation in an oral cavity.

Also provided is said oral care composition of the invention for use in a method for desensitizing hypersensitive teeth by applying thereto a desensitizing amount of the oral composition of the invention.

Also provided is said oral care composition of the invention for use in a method of reducing or inhibiting biofilm formation in an oral cavity, the method comprising contacting the oral cavity with said oral care composition.

### DETAILED DESCRIPTION

As used throughout, ranges are used as shorthand for describing each and every value that is within the range. Any value within the range can be selected as the terminus of the range. Unless otherwise specified, all percentages and amounts expressed herein and elsewhere in the specification should be understood to refer to percentages by weight. The amounts given are based on the active weight of the material.

Open terms such as "include," "including," "contain," "containing" and the like mean "comprising." In this description, unless otherwise stated, the use of the singular also includes the plural. For example, "a zinc ion source" also comprehends the case where more than one zinc ion source is used.

The present inventors have surprisingly found that the combination of (a) a silica abrasive having an average particle size of 8 microns or less; (b) zinc citrate, (c) a bioadhesive agent comprising a polyvinyl methyl ether/maleic anhydride copolymer ("PVM/MA"), and (d) an anticalculus agent comprising tetrapotassium pyrophosphate ("TKPP") and tetrasodium pyrophosphate ("TSPP") improves the efficacy of the oral care composition in reducing biofilm and provides a strong tooth hypersensitivity benefit.

The composition of the invention includes zinc citrate. Zinc ions have been found to help in the reduction of gingivitis, plaque, sensitivity, and improved breath benefits. Additional zinc compounds can be a soluble or sparingly soluble compound of zinc with inorganic or organic counter ions. Examples include the fluoride, chloride, chlorofluoride, acetate, hexafluorozirconate, sulfate, tartrate, gluconate, lactate, malate, glycinate, pyrophosphate, metaphosphate, oxalate, phosphate, carbonate salts, oxides of zinc, and other salts listed in U.S. Pat. No. 4,022,880.

Zinc ions are derived from the zinc compound, i.e., zinc ion source, present in the dentifrice composition in an effective amount. An effective amount of zinc ions is defined as from at least 1000 ppm zinc ion, preferably 2,000 ppm to 15,000 ppm. More preferably, zinc ions are present in an amount from 3,000 ppm to 13,000 ppm and even more preferably from 4,000 ppm to 10,000 ppm. This is the total amount of zinc ions that is present in the compositions for delivery to the tooth surface. The amount of zinc compound (zinc ion source) employed in the oral composition of the invention can vary from 0.01% to 8 wt %, based on the total weight of the composition, typically from 0.1 to 5 %, or 1% to 4%, or 1.5%, based on the total weight of the oral care composition.

According to the invention, the composition contains zinc citrate. In some embodiments, the amount of zinc citrate in the compositions of the invention is 0.01 to 5%, or 0.1 to 5%, or 0.2 to 5%, or 0.2 to 3%, or 1 % to 3 %, based on the total weight of the composition. In some embodiments, the compositions may comprise zinc oxide in an amount of 0.75 % to 1.25 % and zinc citrate in an amount of 0.4 % to 0.6 weight %, based on the total weight of the composition. Optionally, if zinc citrate and zinc oxide are used, a weight ratio or zinc oxide to zinc citrate is 1.5:1 to 4.5:1, 1.5:1 to 4:1, 1.7:1 to 2.3:1, 1.9:1 to 2.1:1, or 2:1. In other embodiments the composition does not contain zinc oxide.

The compositions of the invention comprise a silica abrasive having an average particle size of 8 microns or less. One or more of such silica particles is/are capable of becoming lodged within the tubule, thereby effecting a reduction or elimination of perceived tooth sensitivity. Suitable particles have an average particle size of 8 microns or less, alternatively, 1.5 - 6 microns, 2.7 - 4 microns or 3.5 microns. The particles may be initially present in the composition having the desired particle size, or may be initially present in the composition at a larger size, so long as the structure of the particles is such that it fractures or breaks into the desired particle size upon application of mechanical force by, e.g., a toothbrush, when brushing.

The silica particles may be prepared by any means known or to be developed in the art, and may be surface modified, if desired, to increase the capacity of the particle to adhere to a tooth surface. Examples may be found in, e.g., U.S. patent no. 8,211,452. The silica particles having an average particle size of 8 microns or less may be present in the composition in an amount of 1 to 20% by weight of the total composition. Alternatively, the silica particles having an average particle size of 8 microns or less may be present in an amount of 2 to 10% by weight or 4 to 6% by weight based on the total weight of the composition.

An embodiment of the silica particles having an average particle size of 8 microns or less are commercially available silicas such as PQ Corporation (formerly from INEOS) SORBOSIL AC43 (SORBOSIL AC43 is a silica with properties including a powder RDA of 160, an oil absorption coefficient of 75 cm3/100 g, a weight mean particle size of 3.5 microns, an ignition loss at 1000° C. of 11.0, and a pH of 5.5). Another embodiment is a silica with properties including an average particle size of 2.7-4.0 microns (as determined by MALVERN MASTERSIZER), a sieve residue of +45 µm, a moisture loss at 105° C. of 8.0% max, an ignition loss at 1000° C. of 14.0% max, and a pH of 5.5-7.5 in aqueous suspension), available from Ineos Silicas, Warrington, United Kingdom. Another embodiment of the invention is a silica with an average particle size of 1.5 - 6.0 microns.

The oral care composition may further comprise one or more additional abrasives, i.e., in addition to the silica abrasive that has an average particle size that is no greater than the average diameter of a mammalian dentin tubule. Such additional abrasives can be silica or non-silica. Suitable additional abrasives which may be included in the compositions include, but are not limited to: aluminum oxide, aluminum silicate, calcined alumina, bentonite, other siliceous materials, insoluble phosphates, natural calcium carbonate (NCC), precipitated calcium carbonate (PCC), and mixtures thereof. In some embodiments, at least one of the additional abrasives is a calcium carbonate abrasive, such as precipitated calcium carbonate (PCC) or natural calcium carbonate (NCC).

The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid^{®} by the W. R. Grace & Co., Davison Chemical Division. The precipitated silica materials include those marketed by the J. M. Huber Corp. under the trade name Zeodent^{®}, including the silica carrying the designation Zeodent 115 and 119. These silica abrasives are described in U.S. Pat. No. 4,340,583. In certain embodiments, abrasive materials useful in the practice of the oral compositions in accordance with the invention include silica gels and precipitated amorphous silica having an oil absorption value of less than 100 cc/100 g silica and in the range of 45 cc/100 g to 70 cc/100 g silica. Oil absorption values are measured using the ASTA Rub-Out Method D281. Low oil absorption silica abrasives particularly useful in the practice of the invention are marketed under the trade designation Sylodent XWA^{®} by Davison Chemical Division of W.R. Grace & Co., Baltimore, Md. 21203. Sylodent 650 XWA^{®}, a silica hydrogel composed of particles of colloidal silica having a water content of 29% by weight and an oil absorption of less than 70 cc/100 g of silica is an example of a low oil absorption silica abrasive useful in the practice of the present invention.

The total amount of abrasives in the composition of the invention, i.e., silica(s) plus any additional abrasives, is, in one embodiment, 5 to 60% by weight, in another embodiment 5 to 45% by weight, in another embodiment 5 to 35% by weight, in another embodiment 5 to 30% by weight, in another embodiment 5 to 25 % by weight, in another embodiment 10 to 20% by weight.

The oral care compostions of the invention comprise tetrapotassium pyrophosphate and tetrasodium pyrophosphate as an anticalculus or anti-tartar agent.

The oral care composition also comprises a bioadhesive agent. preferably comprising a polymethyl vinyl ether-maleic anhydride copolymer. In various embodiments, one may use a copolymer of methyl vinyl ether and maleic anhydride, in for example, a monomer ratio of 1:4 to 4:1. Preparation of the polymethyl vinyl ether-maleic anhydride copolymer is specifically set forth in U.S. Pat. No. 5,047,490 to Pelah et al.. The polymethyl vinyl ether maleic anhydride copolymer is also commercially available under the trade name Gantrez^{®} AN from ISP (Ashland), Wayne, N.J. An example of such a polymer comprises:

-[-CH2-CH2OCH3-CH2COOH-CH2COOH-]-

units in its structure.

Such Gantrez^{®} polymers may be linear polymers, or cross linked polymers. Linear, noncross linked, polymers of this type are commercially available under the trade name Gantrez^{®} S (CAS # 25153-4-69), e.g. Gantrez^{®} S-96 having a molecular weight ca. 700,000, Gantrez^{®} S-97 having a molecular weight ca. 1,200,000. Cross linked polymers of this type are also commercially available under the Gantrez^{®} trademark. An example of a derivative of such an acid is an anhydride, i.e. in which the two adjacent -COOH groups are cyclised to form a ring system, such an anhydride is susceptible to hydrolysis to form the corresponding free acids. Such polymers are commercially available under the trade name Gantrez^{®} AN(CAS # 9011-16-9), e.g. Gantrez^{®} AN-119, Gantrez^{®} AN-903, Gantrez^{®} AN-139, Gantrez^{®} AN-169. Another example of a derivative is a partial salt, e.g. where some of the free -COOH groups are converted into a metal salt of a Group I or Group II metal such as respectively either sodium or calcium, or a mixed sodium-calcium salt. Such a polymer is commercially available under the trade name Gantrez^{®} MS, e.g. Gantrez^{®} MS-955 (CAS # 62386-95-2). Another example of a derivative of such an acid is a partial ester in which some of the free -COOH groups are esterified with C1-6 alkyl e.g. ethyl or n-butyl. Such polymers are commercially available under the trade name Gantrez^{®} ES, e.g. Gantrez^{®} ES-225 (CAS # 25087-06-03) or Gantrez^{®} ES-425 (CAS # 25119-68-0. Typically polymers of this second type have molecular weights in the range 200,000-2,000,000.

The compositions further comprise anticalculus (tartar control) agents comprising tetrapotassium pyrophosphate and tetrasodium pyrophosphate. Suitable additional anticalculus agents include, but are not limited to: phosphates and polyphosphates, polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates, sodium tripolyphosphate, tetrapolyphosphate, sodium trimetaphosphate, sodium hexametaphosphate and mixtures thereof. Other useful tartar control agents include polycarboxylate polymers and.

In some embodiments, the compositions may further comprise one or more other bioadhesive agents, i.e., in addition to the polyvinyl methyl ether/maleic anhydride copolymers, such as Gantrez^{®}. The adherent material or bioadhesive agent may be any known or to be developed in the art that attaches to the surface of a mammalian tooth and/or to the heterogenous biofilm which also may be present on a tooth's surface. Attachment may occur by any means, such as ionic interaction, van der Waals forces, hydrophobic-hydrophilic interactions, etc. The adherent material may be, for example, hydrogels, povidone, polysaccharides, chitosan, carrageenans, carboxymethyl cellulose, chitin, gums, or marine colloids. Specific examples of gums include xanthan gum, guar gum, gum arabic, locust bean gum, gum tragacanth, gellan gum and tara gum. Other contemplated adherent materials include any homopolymers or copolymers (hereinafter referred to collectively as a "polymer") that adhere to the surface of a tooth. Such polymers may include polyacrylamides, polyacrylic acid copolymers, polyethylene glycol, cellulose ethers, silicone polymers, polymers having monomers of polyvinyl phosphonic acid, poly(1-phosphonopropene), sulfonic acid, poly(beta styrene phosphonic acid), alpha styrene phosphonic acid, synthetic anionic polymeric polycarboxylate, maleic anhydride, maleic acid, and methyl vinyl ether, poly (ethylene oxide) polymers (such as POLYOX from Dow Chemical), linear polyvinylpyrrolidone (PVP) and cross-linked PVP, PEG/PPG copolymers (such as BASF Pluracare L1220), ester gum, shellac, pressure sensitive silicone adhesives (such as BioPSA from Dow-Corning), methacrylates. Polymers of any molecular weight may be used, including, for example molecular weights 1,000 to 5,000 (number average). Other polymers that may be used as adherent materials include those recited in U.S. Pat. Nos. 4,521,551; 4,485,090; 4,138,477; 4,138,914; and 3,956,480,

In some embodiments, the total amount of bioadhesive agent is present in the composition in an amount of from 0.1 weight % to 10 weight %; 0.5 weight % to 4 weight %; 1 weight % to 2 weight %; or 1.5 weight %, based on the total weight of the composition.

In some embodiments, the polyvinyl methyl ether/maleic anhydride copolymer is present in the composition in an amount of from 0.1 weight % to 10 weight %; 0.5 weight % to 4 weight %; 1 weight % to 2 weight %; or 1.5 weight %, based on the total weight of the composition.

In some embodiments, the total amount of anticalculus agent is present in the composition in an amount of from 0.5 weight % to 5 weight %; 1.0 weight % to 3 weight %; or 2.4 to 2.5 weight %, based on the total weight of the composition.

In some embodiments, the tetrapotassium pyrophosphate and tetrasodium pyrophosphate is present in the composition in an amount of from 0.5 weight % to 5 weight %; 1.0 weight % to 3 weight %; or 2.4 to 2.5 weight %, based on the total weight of the composition.

In another embodiment the composition does not contain a poly(propylene oxide)/poly(ethylene oxide) copolymer, commonly known as a Poloxamer.

In any of the embodiments described herein, the oral care composition may be a dentifrice, a toothpaste, a gel, a tooth powder, a mouthwash, a mouthrinse, a lozenge, a tablet, a spray, a gum, or a film. In certain embodiments, the oral care composition is a toothpaste, a gel, or a tooth powder.

In a second aspect, provided is an oral care composition according to any of the herein embodiments, for use in reducing or inhibiting biofilm formation in an oral cavity.

In a third aspect, provided is a composition for use in a method of reducing or inhibiting biofilm formation in an oral cavity comprising contacting the oral cavity with an oral care composition according to any of the herein embodiments.

Also provided is a composition of the invention for use in a method of reducing dental sensitivity comprising applying to the surface of a mammalian tooth said composition as defined in the claims, wherein the silica abrasive is, e.g., SORBOSIL AC43. In one embodiment the silica particles of the required size are present in the composition in an amount of 5% by weight or greater.

The oral care compositions may further comprise additional ingredients, such as an orally acceptable carrier or base material. These additional ingredients may include, but are not limited to, diluents, bicarbonate salts, pH modifying agents, surfactants, foam modulators, thickening agents, humectants, sweeteners, flavorants, pigments, additional antibacterial agents, anticaries agents, and mixtures thereof.

In some embodiments, the oral care compositions comprise at least one bicarbonate salt useful for example to impart a "clean feel" to teeth and gums due to effervescence and release of carbon dioxide. Any orally acceptable bicarbonate can be used, including without limitation, alkali metal bicarbonates such as sodium and potassium bicarbonates, ammonium bicarbonate and the like. The one or more additional bicarbonate salts are optionally present in a total amount of 0.1 wt. % to 50 wt. %, for example 1 wt. % to 20 wt. %, by total weight of the composition.

In some embodiments, the oral care compositions comprise at least one pH modifying agent. Such agents include acidifying agents to lower pH, basifying agents to raise pH, and buffering agents to control pH within a desired range. For example, one or more compounds selected from acidifying, basifying and buffering agents can be included to provide a pH of 2 to 10, or in various illustrative embodiments, 2 to 8, 3 to 9, 4 to 8, 5 to 7, 6 to 10, 7 to 9, etc. Any orally acceptable pH modifying agent can be used, including without limitation, carboxylic, phosphoric and sulfonic acids, acid salts (e.g., monosodium citrate, disodium citrate, monosodium malate, etc.), alkali metal hydroxides such as sodium hydroxide, carbonates such as sodium carbonate, bicarbonates, sesquicarbonates, borates, silicates, phosphates (e.g., monosodium phosphate, trisodium phosphate), imidazole and the like. One or more pH modifying agents are optionally present in a total amount effective to maintain the composition in an orally acceptable pH range.

The oral care compositions may also comprise at least one surfactant. Any orally acceptable surfactant, most of which are anionic, nonionic or amphoteric, can be used. Suitable anionic surfactants include without limitation, water-soluble salts of C8-20 alkyl sulfates, sulfonated monoglycerides of C8-20 fatty acids, sarcosinates, taurates and the like. Illustrative examples of these and other classes include sodium lauryl sulfate (SLS), sodium coconut monoglyceride sulfonate, sodium lauryl sarcosinate, sodium lauryl isoethionate, sodium laureth carboxylate and sodium dodecyl benzenesulfonate. Suitable nonionic surfactants include without limitation, poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation, derivatives of C8-20 aliphatic secondary and tertiary amines having an anionic group such as carboxylate, sulfate, sulfonate, phosphate or phosphonate. Betaines may also be used, a suitable example of which is cocoamidopropyl betaine. One or more surfactants are optionally present in a total amount of 0.01 wt.% to 10 wt. %, for example, from 0.05 wt. % to 5 wt. %, or from 0.1 wt. % to 2 wt. % by total weight of the composition.

The oral care compositions may comprise at least one foam modulator, useful for example to increase amount, thickness or stability of foam generated by the composition upon agitation. Any orally acceptable foam modulator can be used, including without limitation, polyethylene glycols (PEGs), also known as polyoxyethylenes. High molecular weight PEGs are suitable, including those having an average molecular weight of 200,000 to 7,000,000, for example 500,000 to 5,000,000, or 1,000,000 to 2,500,000. One or more PEGs are optionally present in a total amount of 0.1 wt. % to 10 wt. %, for example from 0.2 wt. % to 5 wt. %, or from 0.25 wt. % to 2 wt.%, by total weight of the composition.

The oral care compositions may comprise at least one thickening agent, useful for example to impart a desired consistency and/or mouth feel to the composition. Any orally acceptable thickening agent can be used, including without limitation, carbomers, also known as carboxyvinyl polymers, carrageenans, also known as Irish moss and more particularly τ-carrageenan (iotacarrageenan), cellulosic polymers such as hydroxyethylcellulose, carboxymethylcellulose (CMC) and salts thereof, e.g., CMC sodium, natural gums such as karaya, xanthan, gum arabic and tragacanth, colloidal magnesium aluminum silicate, colloidal silica and the like. A preferred class of thickening or gelling agents includes a class of homopolymers of acrylic acid crosslinked with an alkyl ether of pentaerythritol or an alkyl ether of sucrose, or carbomers. Carbomers are commercially available from B. F. Goodrich as the Carbopol^{®} series. Particularly preferred Carbopols include Carbopol 934, 940, 941, 956, 974P, and mixtures thereof. Silica thickeners such as DT 267 (from PPG Industries) may also be used. One or more thickening agents are optionally present in a total amount of from 0.1 wt. % to 15 wt.%, for example from 0.5 wt.% to 10 wt.%, or from 1 wt. % to 5 wt.%, by total weight of the composition.

The compositions may comprise at least one viscosity modifier, useful for example to help inhibit settling or separation of ingredients or to promote re-dispersibility upon agitation of a liquid composition. Any orally acceptable viscosity modifier can be used, including without limitation, mineral oil, petrolatum, clays and organomodified clays, silicas and the like. One or more viscosity modifiers are optionally present in a total amount of from 0.01 wt. % to 10 wt. %, for example, from 0.1 wt.% to 5 wt.%, by total weight of the composition.

The compositions may also comprise at least one humectant. Any orally acceptable humectant can be used, including without limitation, polyhydric alcohols such as glycerin, sorbitol (optionally as a 70 wt.% solution in water), xylitol or low molecular weight polyethylene glycols (PEGs). Most humectants also function as sweeteners. One or more humectants are optionally present in a total amount of from 1 wt.% to 70 wt.%, for example, from 1 wt.% to 50 wt.%, from 2 wt.% to 25 wt.%, or from 5 wt.% to 15 wt.%, by total weight of the composition.

The oral care compositions may comprise at least one sweetener, useful for example to enhance taste of the composition. One or more sweeteners are optionally present in a total amount depending strongly on the particular sweetener(s) selected, but typically 0.005 wt.% to 5 wt.%, by total weight of the composition, optionally 0.005 wt.% to 0.2 wt.%, further optionally 0.05 wt.% to 0.1 wt.% by total weight of the composition.

The compositions may also comprise at least one flavorant, useful for example to enhance taste of the composition. Any orally acceptable natural or synthetic flavorant can be used, including without limitation tea flavours, vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or warming effects. Such ingredients illustratively include menthol, menthyl acetate, menthyl lactate, camphor, eucalyptus oil, eucalyptol, anethole, eugenol, cassia, oxanone, α-irisone, propenyl guaiethol, thymol, linalool, benzaldehyde, cinnamaldehyde, N-ethyl-p-menthan-3-carboxamine, N,2,3-trimethyl-2-isopropylbutanamide, 3-(1-menthoxy)-propane-1,2-diol, cinnamaldehyde glycerol acetal (CGA), menthone glycerol acetal (MGA) and the like. One or more flavorants are optionally present in a total amount of from 0.01 wt. % to 5 wt. %, for example, from 0.03 wt. % to 2.5 wt.%, optionally 0.05 wt.% to 1.5 wt.%, further optionally 0.1 wt.% to 0.3 wt.% by total weight of the composition.

The compositions may comprise at least one colorant. Colorants herein include pigments, dyes, lakes and agents imparting a particular luster or reflectivity such as pearling agents. Any orally acceptable colorant can be used, including without limitation talc, mica, magnesium carbonate, magnesium silicate, magnesium aluminum silicate, titanium dioxide, red, yellow, brown and black iron oxides, ferric ammonium ferrocyanide, manganese violet, ultramarine, titaniated mica, bismuth oxychloride, and the like. One or more colorants are optionally present in a total amount of from 0.001 wt.% to 20 wt.%, for example, from 0.01 wt.% to 10 wt. %, or from 0.1 wt. % to 5 wt.%, by total weight of the composition.

The compositions may also comprise an additional antibacterial or preservative agent, such as chlorhexidine, triclosan, quaternary ammonium compounds (for example benzalkonium chloride) or parabens such as methylparaben or propylparaben. One or more additional antibacterial or preservative agents may optionally be present in the composition in a total amount of from 0.01 wt.% to 0.5 wt.%, optionally 0.05 wt.% to 0.1 wt.% by total weight of the composition.

The oral care compositions may also comprise a fluoride ion source. Fluoride ion sources include, but are not limited to: stannous fluoride, sodium fluoride, potassium fluoride, potassium monofluorophosphate, sodium monofluorophosphate, ammonium monofluorophosphate, sodium fluorosilicate, ammonium fluorosilicate, amine fluoride such as olaflur (N'octadecyltrimethylendiamine-N,N,N'-tris(2-ethanol)-dihydrofluoride), ammonium fluoride, and combinations thereof. In certain embodiments the fluoride ion source includes stannous fluoride, sodium fluoride, amine fluorides, sodium monofluorophosphate, as well as mixtures thereof. In certain embodiments, the oral care composition may also contain a source of fluoride ions or fluorine-providing ingredient in amounts sufficient to supply 50 to 5000 ppm fluoride ion, e.g., from 100 to 1000, from 200 to 500, or 250 ppm fluoride ion. Fluoride ion sources may be added to the compositions at a level of 0.001 wt. % to 10 wt. %, e.g., from 0.003 wt. % to 5 wt. %, 0.01 wt. % to 1 wt., or 0.05 wt. %. However, it is to be understood that the weights of fluoride salts to provide the appropriate level of fluoride ion will obviously vary based on the weight of the counter ion in the salt, and one of skill in the art may readily determine such amounts. A preferred fluoride salt may be sodium fluoride.

The compositions may comprise a saliva stimulating agent useful, for example, in amelioration of dry mouth. Any orally acceptable saliva stimulating agent can be used, including without limitation food acids such as citric, lactic, malic, succinic, ascorbic, adipic, fumaric and tartaric acids, and mixtures thereof. One or more saliva stimulating agents are optionally present in saliva stimulating effective total amount.

The compositions may include antisensitivity agents, e.g., potassium salts such as potassium nitrate, potassium bicarbonate, potassium chloride, potassium citrate, and potassium oxalate; capsaicin; eugenol; strontium salts; chloride salts and combinations thereof Such agents may be added in effective amounts, e.g., from 1 wt. % to 20 wt. % by weight based on the total weight of the composition, depending on the agent chosen.

The composition may further comprise an antioxidant. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

In some embodiments, the compositions of the present invention further comprise an amino acid. In some embodiments, the amino acid is present in a desensitizing effective amount. In some embodiments, the amino acid comprises from 0.01% to 10%, by weight, of the composition. In some embodiments, the amino acid comprises from 0.1% to 7%, by weight, of the composition. In some embodiments, the amino acid comprises from 0.5% to 5%, by weight, of the composition. In some embodiments, the amino acid comprises from 1% to 4%, by weight, of the composition. In some embodiments, the amino acid comprises from 2% to 3%, by weight, of the composition. In some embodiments, the amino acid comprises 2.5%, by weight, of the composition. In some embodiments, the amino acid comprises arginine. In some embodiments, the amino acid comprises L-arginine and/or L-serine. In some embodiments, the amino acid comprises L-arginine bicarbonate. In some embodiments, L-arginine bicarbonate comprises 2.5%, by weight, of the composition. In some embodiments the composition does not contain an amino acid, e.g., a basic amino acid such as arginine.

It is understood that while general attributes of each of the above categories of materials may differ, there may be some common attributes and any given material may serve multiple purposes within two or more of such categories of materials. All of the ingredients in the compositions may have functions in addition to their primary function, and may contribute to the overall properties of the composition, including its stability, efficacy, consistency, mouthfeel, taste, odor and so forth. Preferably, the ingredients are selected for compatibility with other ingredients of the composition.

Also provided are compositions for use in a method to reduce and inhibit acid erosion of the enamel, clean the teeth, reduce bacterially-generated biofilm and plaque, reduce gingivitis, inhibit tooth decay and formation of cavities, and reduce dentinal hypersensitivity, comprising applying an effective amount of a composition, e.g., any of Composition 1, *et seq.* to the teeth.

Also provided is a method of making an oral care composition comprising (a) a silica abrasive having an average particle size of no greater than the diameter of a dentin tubule ; (b) zinc citrate, (c) a bioadhesive agent preferably comprising a polyvinyl methyl ether/maleic anhydride ("PVM/MA") copolymer, and (d) an anticalculus agent preferably tetrapotassium pyrophosphate ("TKPP") e.g., any of Composition 1, *et seq.* comprising combining components (a), (b), (c) and (d) with an oral care base, e.g., a dentifrice or mouthwash base.

For example, in various embodiments, provided are compositions for use in methods to (i) reduce hypersensitivity of the teeth, (ii) to reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; (xv) reduce tartar build-up, and/or (xvi) promote systemic health, including cardiovascular health, e.g., by reducing potential for systemic infection via the oral tissues, comprising applying any of Compositions 1, *et seq.* as described above to the oral cavity of a person in need thereof, e.g., one or more times per day. Also provided are Compositions 1, *et seq.* for use in any of these methods.

Also provided is the use of (a) a silica abrasive having an average particle size of no greater than the diameter of a dentin tubule; (b) zinc citrate, and (c) a bioadhesive agent preferably comprising a polyvinyl methyl ether/maleic anhydride ("PVM/MA") copolymer, and (d) an anticalculus agent preferably comprising tetrapotassium pyrophosphate ("TKPP") in the manufacture of an oral care composition, e.g., in accordance with any of Compositions 1, *et seq..*

Also provided is an oral care composition comprising:(a) a silica abrasive having an average particle size of 8 microns or less;(b) zinc citrate,(c) a bioadhesive agent comprising a polyvinyl methyl ether/maleic anhydride copolymer, and(d) an anti calculus agent comprising tetrapotassium pyrophosphate and tetrasodium pyrophosphate for use in a method to reduce and inhibit acid erosion of the enamel, clean the teeth, reduce bacterially-generated biofilm and plaque, reduce gingivitis, inhibit tooth decay and formation of cavities, and/or reduce dentinal hypersensitivity.

A type of product form is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

Another type of product form is a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated. A mouthwash composition will usually contain an aqueous continuous phase. The amount of water generally ranges from 70 to 99% by weight based on the total weight of the mouthwash.

Classification or discussion of a material within a section of this specification as having a particular utility (e.g., as being an "active" or a "carrier" ingredient) is made for convenience, and no inference should be drawn that the material must necessarily or solely function in accordance with its classification herein when it is used in any given composition.

The invention is illustrated by the following non-limiting examples.

### EXAMPLES

### Example 1

Formula A (not claimed) is a toothpaste composition of the invention and Formula B is a control toothpaste composition. Formula C (not claimed) is similar to Formula A except that it contains 2% TKPP, 1% TSPP, and no Gantrez. Formula D (not claimed) is similar to Formula A except that it contains 1% TKPP, 1% TSPP, and no Gantrez. Formula E (not claimed) is similar to Formula A except that it contains no zinc.

**Formula A**

| INGREDIENTS | WEIGHT PERCENT |
|---|---|
| Polyethylene Glycol 600 | 3.00 |
| Sodium CMC | 0.55 |
| Gantrez S-97 (B.F.) Powder | 1.50 |
| Sodium Saccharin | 0.25 |
| Sodium Monofluorophosphate | 1.10 |
| Sorbitol - Non-Crystal - 70% Soln | 21.52 |
| Glycerin | 11.50 |
| Zinc Citrate Trihydrate | 2.00 |
| Tetrapotassium Pyrophosphate | 2.44 |
| Sodium Hydroxide 50% | 1.60 |
| Demineralized Water | 28.04 |
| Syn. Amorph. Ppt. Silica- Abrasive | 15.00 |
| Syn. Amorph. Ppt. Silica-Thickener | 2.00 |
| Flavor | 1.30 |
| 95% Na Lauryl Sulfate Granules | 2.30 |
| Titanium Dioxide | 0.50 |
| Titanium Dioxide Coated Mica | 0.20 |
| Xanthan Gum | 0.20 |
| Silica (SORBOSIL AC43 silica) | 5.00 |
| TOTAL | 100.00 |

**Formula B**

| INGREDIENTS | WEIGHT PERCENT |
|---|---|
| Sorbitol - Non-Crystal - 70% Soln | 31.00 |
| Sodium CMC | 0.80 |
| Sodium Saccharin | 0.18 |
| Sodium Monofluorophosphate | 0.76 |
| Sodium Fluoride | 0.10 |
| Tetrasodium Pyrophosphate | 0.25 |
| Demineralized Water | 18.81 |
| Dicalcium Phosphate Dihydrate | 45.00 |
| Flavor | 1.10 |
| 95% Na Lauryl Sulfate Granules | 2.00 |
| TOTAL | 100.00 |

Another embodiment of the composition is a depicted in the table below:

| Ingredients | % |
|---|---|
| Sorbitol | 10-60%, e.g., 15-30% |
| Polyvinyl methyl ethcr/maleic anhydride (PVM/MA) copolymer | 1-3%, e.g., 1.5% |
| Zinc citrate | 0.5-3%, e.g., 1.5% or 2%% |
| Carboxymethyl cellulose (CMC) | 0.1-1%, e.g., 0.55% |
| Flavoring and/or sweetener | 0.01-1% |
| Propylene Glycol | 1-5%, e.g., 3% |
| Silica, average diameter of 8µ or less | 1-15%, e.g., 5 % |
| Tetrapotassium pyrophosphate (TKPP) | 0.5-3%, e.g., 1-3%, e.g., 2.44% |
| Sodium monofluorophosphate | 0.5-3%, e.g., 1.1% |
| Glycerin | 5 -25%, e.g., 11.5% |
| Sodium lauryl sulfate (SLS) | 0.5-5%, e.g., 2.3% |

### Example 2

Experiments were carried out in order to evaluate the biofilm reduction efficacy of toothpaste compositions.

In this Example, the experimental methodology used was the Biofilm Growth Inhibition University of Manchester Model using standard toothpaste formulations with key ingredients specified in the tables below. The protocol for this model is as follows:
(1) Dental plaque was collected from four healthy volunteers and pooled together as inoculum.
   The Optical Density of the inoculum was matched to 0.3 absorbance at 610nm
(2) Sterile hydroxyapatite (HAP) disks were incubated under anaerobic conditions at 37°C for 24 hours with 1mL of sterile artificial saliva (with 0.01 weight% sucrose) and 1mL of pooled saliva in a 24 well microplate.
(3) For each test dentifrice (and for each control) a treatment solution of 1 part dentifrice: 2 parts sterile distilled water by weight was made up. Each freshly prepared treatment solution was added to three wells and allowed to contact the HAP disk therein for 10 minutes.
(4) The liquid phase of each well was then removed and was replaced by 2mL sterile artificial saliva.
(5) The disks were then maintained at 37°C under anaerobic conditions for 8 days.
(6) At intervals of 2, 4 and 8 days, the disks were collected aseptically and transferred to halfstrength pre-reduced thioglycollate medium (4.5 ml per disk).
(7) 100µL of the dilution 10-4, 10-5 and 10-6 were plated in duplicates for each disk on Neomycin/Vancomycin (NV) Agar for Total Gram-negative Anaerobes.
(8) The plates were surface-spread using a sterile spreader and were incubated anaerobically at 37°C for 72 hours, after which time the number of colonies on each plate was counted.

The log10 CFU/ml (where CFU = colony forming units) for each test dentifrice or control was calculated. A lower Log10 CFU/ml indicates that the dentifrice tested has greater efficacy in inhibiting biofilm growth.

The results of the tests are shown in the tables, below.

**Table A1. Biofilm reduction. The table is ranked in descending order of efficacy**

| No | Formula | Avg. Log |
|---|---|---|
| 1 | Formula A: 5% Silica (AC43), 2% ZnCit, 1.5% Gantrez, 2.44% TKPP | 5.74 |
| 2 | Formula C: 5% Silica (AC43), 2% ZnCit, 2% TKPP, 1% TSPP | 6.02 |
| 3 | Formula D: 5%Silica (AC43), 2% ZnCit,, 1% TKPP, 1% TSPP | 6.19 |
| 4 | Competitive dentifrice 1 (Crest Pro-Health High water): silica, 0.64% SnCl2, 0.54% ZnCit) | 6.38 |
| 5 | Formula E: 5% Silica (AC43), no Zinc (Placebo) | 6.46 |

**Table A2. Biofilm reduction. The table is ranked in descending order of efficacy**

| No | Formula | Avg. Log |
|---|---|---|
| 1 | Silica, 0.3% Triclosan, 2% Gantrez | 4.99 |
| 2 | Silica, no Zinc (Placebo) | 6.27 |
| 3 | Competitive dentifrice 1 (Crest Pro Health High water): silica, 0.64% SnCl2, 0.54% ZnCit | 6.28 |

### Example 3

In this Example, the experimental methodology used was the artificial mouth method using standard toothpaste formulations with key ingredients specified in the tables below. The Artificial Mouth method protocol is described below:
1. Saliva coated HAP disks are placed in the flow cell and connected to a one flow medium passage.
2. The medium flows from the reservoir through the flow cells and gets remove to the waste.
3. Untreated sample is used as a control to test the efficacy of the dentrifices
4. Treatment solutions of 1:2 diluted dentifrices (10 ml) were then drawn into the flow cells to contact the disks.
5. The flow of media was reinstated to rinse the treatment and further allow the growth of biofilm during the study period.
6. The treatment was repeated over a time interval of 24 hours with the flow adjusted to stimulate conditions in the human mouth.
7. The HAP disks were collected at the end of the study and introduced into anaerobic media, vortex to make the plaque on disc thoroughly suspend in the solution
8. Pipet out 300µl of the suspension into the wells of a 96-well microplate, read optical density (OD) @ 630nm in the microplate reader.
9. Use an EXCEL spreadsheet to calculate means and standard deviation of the different treatments. The lower the OD, the better the efficacy of the treatment.
10. The suspension from the flowcell is collected and diluted serially with anaerobic transfer media
11. Serial dilutions are plated on lead acetate plates and incubated anaerobically @37degree C for 2-3 days.
12. Calculate the real colony forming units on the HAP disc and report the final result as Log cfu/disc. The lower the value, the better the efficacy of the treatment.

The results are in the tables below.

**Table A3. Artificial Mouth OD value at 610 nm.**

| Lower numbers represent higher efficacy against plaque | | |
|---|---|---|
| **No** | **Formula** | **Mean OD** |
| 1 | Formula A: Silica, 2% ZnCitrate, 1.5% Gantrez, 2.44% TKPP | 0.6278 |
| 2 | Control: chalk, no Zinc | 1.4322 |
| 3 | Competitive dentifrice 2 (Crest Pro-Health Low Water): silica, 2% ZnLactate, SnF2 | 1.1667 |

**Table A4. Artificial Mouth plate count on NV agar**

| **No** | **Formula** | **Mean OD** |
|---|---|---|
| 1 | Formula A: AC43 Silica, 2% ZnCitrate, 1.5% Gantrez, 2.44% TKPP | 6.0800 |
| 2 | Control: chalk, no Zinc | 7.4417 |
| 3 | Competitive dentifrice 2 (Crest Pro-Health Low Water): silica, 2% ZnLactate, SnF2 | 6.726 |

**Table A5. Artificial Mouth plate count on PbAc agar**

| **No** | **Formula** | **Mean OD** |
|---|---|---|
| 1 | Formula A: AC43 Silica, 2% ZnCitrate, 1.5% Gantrez, 2.44% TKPP | 5.4939 |
| 2 | Control: chalk, no Zinc | 6.2840 |
| 3 | Competitive dentifrice 2 (Crest Pro-Health Low Water): silica, 2% ZnLactate, SnF2 | 5.6336 |

### Example 4

A clinical study is carried out to compare tooth sensitivity effectiveness of a toothpaste of the invention (Formula A) with a control toothpaste (Formula B). Tactile and air blast hypersensitivities are carried out on two treatment groups, one brushing daily with Formula a and one brushing daily with Formula B. Tactile and air blast hypersensitivities are determined at 0, 2, 4 and 8 weeks.

### Results:

### Baseline - Tactile and Air Blast Hypersensitivity

There was no statistically significant difference between the two treatment groups in baseline mean hypersensitivity scores (tactile and air blast).

After Two Weeks of Brushing - Tactile and Air Blast Hypersensitivity

### Between group comparison

There was no statistically significant difference between the two treatment groups in twoweek mean hypersensitivity scores (tactile and air blast).

After Four Weeks of Brushing - Tactile and Air Blast Hypersensitivity

### Between group comparison

Relative to the group brushing with Formula B, the Formula A group exhibited a statistically significant improvement in mean tactile and air blast hypersensitivity scores after four weeks of product use (23.50% and 21.39%, respectively).

### After Eight Weeks of Brushing - Tactile and Air Blast Hypersensitivity

### Between group comparison

Relative to the group brushing with Formula B, the Formula A group exhibited a statistically significant improvement in mean tactile and air blast hypersensitivity scores after eight weeks of product use (41.18% and 27.69%, respectively).

### Within Treatment:

The Formula A group provided significantly more hypersensitivity reduction in tactile and air blast at all post-baseline measurements (2, 4 and 8 weeks) than at baseline.

The Formula A group provided significantly more hypersensitivity reduction in tactile and air blast at all post-baseline measurements (2, 4 and 8 weeks) than at baseline.

**Table A6. Summary of Tactile Sensitivity Scores (grams of force with Yeaple Probe)**

| Dentifrice Group | N | Baseline Mean ± SD | 2 Week Mean ± SD | 4 Weeks Mean ± SD | 8 Weeks Mean ± SD |
|---|---|---|---|---|---|
| Formula A (2% ZnCitrate and 5% AC43) | 40 | 11.63±2.63 | 21.38±7.93 | 25.75±7.47 | 32.88±7.92 |
| Formula B | 41 | 11.34±2.96 | 19.27±6.48 | 20.85±4.86 | 23.29±7.47 |

**Table A7. Summary of Air Blast Sensitivity Scores (Schiff Thermal Scale)**

| Dentifrice Group | N | Baseline Mean ± SD | 2 Week Mean ± SD | 4 Weeks Mean ± SD | 8 Weeks Mean ± SD |
|---|---|---|---|---|---|
| Formula A (2% ZnCitrate and 5% AC43) | 40 | 2.29±0.39 | 1.84±0.33 | 1.58±0.40 | 1.41±0.54 |
| Colgate Cavity Protection Toothpaste | 41 | 2.29±0.38 | 2.01±0.55 | 2.01±0.55 | 1.95±0.52 |

## Claims

1. An oral care composition comprising:
(a) a silica abrasive having an average particle size of 8 microns or less;
(b) zinc citrate,
(c) a bioadhesive agent comprising a polyvinyl methyl ether/maleic anhydride copolymer, and
(d) an anti calculus agent comprising tetrapotassium pyrophosphate and tetrasodium pyrophosphate.

2. The oral care composition of claim 1, wherein the silica abrasive has an average particle size of 2.7 - 4 microns or 3.5 microns.

3. The oral care composition of any preceding claim, wherein the bioadhesive agent is in an amount of 1.5 weight %, based on the total weight of the composition.

4. The oral care composition of any preceding claim, wherein the anticalculus agent is in an amount of 2.4 to 2.5 weight %, based on the total weight of the composition.

5. The oral care composition of any preceding claim, wherein the total amount of abrasives is 5 to 60% by weight, 5 to 45% by weight, 5 to 35% by weight, 5 to 30% by weight, 5 to 25 % by weight, 10 to 20% by weight, or 20% by weight based on the total weight of the composition.

6. The oral care composition of any preceding claim, wherein the total amount of bioadhesive agent is a polyvinyl methyl ether/maleic anhydride copolymer in amount of 1.5 weight %, based on the total weight of the composition, and
the anticalculus agent is tetrapotassium pyrophosphate and tetrasodium pyrophosphate in an amount of 2.4 to 2.5 weight %, based on the total weight of the composition.

7. The oral care composition of any preceding claim further comprising one or more surfactants selected from anionic, cationic, zwitterionic, and nonionic surfactants, and mixtures thereof.

8. The oral care composition of any preceding claim further comprising a humectant selected from glycerin, sorbitol, propylene glycol, polyethylene glycol, xylitol, and mixtures thereof.

9. The oral care composition of any preceding claim further comprising a viscosity modifying amount of one or more polymers selected from polyethylene glycols, polysaccharides, cellulose derivatives, carboxymethyl cellulose, polysaccharide gums, xanthan gum, carrageenan gum, and combinations thereof.

10. The oral care composition of any preceding claim further comprising a buffering agent, a flavorant, a colorant, a fragrance, a whitening agent or a combination thereof.

11. The oral care composition of any preceding claim further comprising an effective amount of one or more antibacterial agents selected from the group consisting of halogenated diphenyl ether, triclosan, herbal extracts, essential oils, rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract, bisguanide antiseptics, chlorhexidine, alexidine or octenidine, quaternary ammonium compounds, cetylpyridinium chloride (CPC), benzalkonium chloride, tetradecylpyridinium chloride (TPC), N-tetradecyl-4-ethylpyridinium chloride (TDEPC), phenolic antiseptics, hexetidine, octenidine, sanguinarine, povidone iodine, delmopinol, salifluor, stannous salts, copper salts, iron salts, sanguinarine, propolis and oxygenating agents, hydrogen peroxide, buffered sodium peroxyborate or peroxycarbonate, phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts, and mixtures of any of the foregoing.

12. An oral care composition according to any preceding claim, for use in reducing or inhibiting biofilm formation in an oral cavity, or for use in reducing or inhibiting dentinal hypersensitivity.

13. An oral care composition according to any preceding claim, wherein the composition comprises a fluoride source, and wherein the fluoride source is a salt selected from stannous fluoride, sodium fluoride, potassium fluoride, sodium fluorosilicate, ammonium fluorosilicate, amine fluoride, preferably N'octadecyltrimethylendiamine-N,N,N'- tris(2-ethanol)-dihydrofluoride; ammonium fluoride, titanium fluoride, hexafluorosulfate, and combinations thereof.

## Patentansprüche

1. Mundpflegezusammensetzung, umfassend:
(a) ein Silica-Abrasivmittel mit einer durchschnittlichen Teilchengröße von 8 Mikrometern oder weniger;
(b) Zinkcitrat,
(c) ein bioadhäsives Mittel, das ein Polyvinylmethylether/Maleinsäureanhydrid-Copolymer umfasst, und
(d) ein Anti-Zahnstein-Mittel, das Tetrapotassium-Pyrophosphat und Tetranatrium-Pyrophosphat umfasst.

2. Mundpflegezusammensetzung nach Anspruch 1, wobei das Silica-Abrasivmittel eine durchschnittliche Teilchengröße von 2,7 - 4 Mikrometern oder 3,5 Mikrometern aufweist.

3. Mundpflegezusammensetzung nach einem beliebigen vorhergehenden Anspruch, wobei das bioadhäsive Mittel in einer Menge von 1,5 Gewichts-%, bezogen auf das Gesamtgewicht der Zusammensetzung, vorliegt.

4. Mundpflegezusammensetzung nach einem beliebigen vorhergehenden Anspruch, wobei das Anti-Zahnstein-Mittel in einer Menge von 2,4 bis 2,5 Gewichts%, bezogen auf das Gesamtgewicht der Zusammensetzung, vorliegt.

5. Mundpflegezusammensetzung nach einem beliebigen vorhergehenden Anspruch, wobei die Gesamtmenge an Abrasivmitteln 5 bis 60 Gewichts-%, 5 bis 45 Gewichts-%, 5 bis 35 Gewichts-%, 5 bis 30 Gewichts-%, 5 bis 25 Gewichts-%, 10 bis 20 Gewichts-% oder 20 Gewichts-%, bezogen auf das Gesamtgewicht der Zusammensetzung, beträgt.

6. Mundpflegezusammensetzung nach einem beliebigen vorhergehenden Anspruch, wobei die Gesamtmenge des bioadhäsiven Mittels ein Polyvinylmethylether/Maleinsäureanhydrid-Copolymer in einer Menge von 1,5 Gewichts-% ist, bezogen auf das Gesamtgewicht der Zusammensetzung, und
das Anti-Zahnstein-Mittel Tetrapotassiumpyrophosphat und Tetranatriumpyrophosphat in einer Menge von 2,4 bis 2,5 Gewichts-%, bezogen auf das Gesamtgewicht der Zusammensetzung, ist.

7. Mundpflegezusammensetzung nach einem beliebigen vorhergehenden Anspruch, weiterhin umfassend ein oder mehrere oberflächenaktive Mittel, ausgewählt aus anionischen, kationischen, zwitterionischen und nichtionischen oberflächenaktiven Mitteln und Mischungen davon.

8. Mundpflegezusammensetzung nach einem beliebigen vorhergehenden Anspruch, weiterhin umfassend ein Feuchthaltemittel, ausgewählt aus Glycerin, Sorbitol, Propylenglycol, Polyethylenglycol, Xylitol und Mischungen davon.

9. Mundpflegezusammensetzung nach einem beliebigen vorhergehenden Anspruch, weiterhin umfassend eine viskositätsmodifizierende Menge eines oder mehrerer Polymere, ausgewählt aus Polyethylenglykolen, Polysacchariden, Cellulosederivaten, Carboxymethylcellulose, Polysaccharidgummis, Xanthangummi, Carrageenangummi und Kombinationen davon.

10. Mundpflegezusammensetzung nach einem beliebigen vorhergehenden Anspruch, weiterhin umfassend ein Puffermittel, einen Geschmacksstoff, einen Farbstoff, einen Duftstoff, ein Bleichmittel oder eine Kombination davon.

11. Mundpflegezusammensetzung nach einem beliebigen vorhergehenden Anspruch, weiterhin umfassend eine wirksame Menge eines oder mehrerer antibakterieller Mittel, ausgewählt aus der Gruppe bestehend aus halogeniertem Diphenylether, Triclosan, Kräuterextrakten, ätherischen Ölen, Rosmarinextrakt, Teeextrakt, Magnolienextrakt, Thymol, Menthol, Eukalyptol, Geraniol, Carvacrol, Citral, Hinokitol, Catechol, Methylsalicylat, Epigallocatechingallat, Epigallocatechin, Gallussäure, Miswak-Extrakt, Sanddorn-Extrakt, Bisguanid-Antiseptika, Chlorhexidin, Alexidin oder Octenidin, quaternäre Ammoniumverbindungen, Cetylpyridiniumchlorid (CPC), Benzalkoniumchlorid, Tetradecylpyridiniumchlorid (TPC), N-Tetradecyl-4-ethylpyridiniumchlorid (TDEPC), phenolische Antiseptika, Hexetidin, Octenidin, Sanguinarin, Povidon-Jod, Delmopinol, Salifluor, Zinnsalze, Kupfersalze, Eisensalze, Sanguinarin, Propolis und Oxygenierungsmittel, Wasserstoffperoxid, gepuffertes Natriumperoxyborat oder Peroxycarbonat, Phthalsäure und ihre Salze, Monoperthalsäure und ihre Salze und Ester, Ascorbylstearat, Oleoylsarkosin, Alkylsulfat, Dioctylsulfosuccinat, Salicylanilid, Domiphenbromid, Delmopinol, Octapinol und andere Piperidinderivate, Nicin-Zubereitungen, Chloritsalze und Mischungen aus beliebigen der vorhergehenden.

12. Mundpflegezusammensetzung nach einem beliebigen vorhergehenden Anspruch zur Verwendung bei der Reduzierung oder Hemmung von Biofilmbildung in einer Mundhöhle oder zur Verwendung bei der Reduzierung oder Hemmung von dentiner Hypersensitivität.

13. Mundpflegezusammensetzung nach einem beliebigen vorhergehenden Anspruch, wobei die Zusammensetzung eine Fluoridquelle umfasst und wobei die Fluoridquelle ein Salz ist, das ausgewählt ist aus Zinnfluorid, Natriumfluorid, Kaliumfluorid, Natriumfluorosilikat, Ammoniumfluorosilikat, Aminfluorid, vorzugsweise N'-Octadecyltrimethylendiamin-N,N,N'-tris(2-ethanol)-dihydrofluorid; Ammoniumfluorid, Titanfluorid, Hexafluorosulfat und Kombinationen davon.

## Revendications

1. Composition de soins bucco-dentaires comprenant :
(a) un abrasif à base de silice ayant une taille moyenne de particule de 8 microns ou moins ;
(b) du citrate de zinc,
(c) un agent bioadhésif comprenant un copolymère polyvinyl méthyl éther/anhydride maléique, et
(d) un agent antitartre comprenant du pyrophosphate tétrapotassique et du pyrophosphate tétrasodique.

2. Composition de soins bucco-dentaires selon la revendication 1, dans laquelle l'abrasif à base de silice a une taille moyenne de particule de 2,7 à 4 microns ou de 3,5 microns.

3. Composition de soins bucco-dentaires selon l'une quelconque des revendications précédentes, dans laquelle l'agent bioadhésif est présent en une quantité de 1,5 % en poids, par rapport au poids total de la composition.

4. Composition de soins bucco-dentaires selon l'une quelconque des revendications précédentes, dans laquelle l'agent antitartre est présent en une quantité de 2,4 à 2,5 % en poids, par rapport au poids total de la composition.

5. Composition de soins bucco-dentaires selon l'une quelconque des revendications précédentes, dans laquelle la quantité totale d'abrasifs est de 5 à 60 % en poids, de 5 à 45 % en poids, de 5 à 35 % en poids, de 5 à 30 % en poids, de 5 à 25 % en poids, de 10 à 20 % en poids, ou de 20 % en poids par rapport au poids total de la composition.

6. Composition de soins bucco-dentaires selon l'une quelconque des revendications précédentes, dans laquelle la quantité totale d'agent bioadhésif est un copolymère polyvinyl méthyl éther/anhydride maléique en une quantité de 1,5 % en poids, par rapport au poids total de la composition, et
l'agent antitartre est du pyrophosphate tétrapotassique et du pyrophosphate tétrasodique en une quantité de 2,4 à 2,5 % en poids, par rapport au poids total de la composition.

7. Composition de soins bucco-dentaires selon l'une quelconque des revendications précédentes comprenant en outre un ou plusieurs tensioactifs choisis parmi les tensioactifs anioniques, cationiques, zwitterioniques et non ioniques, et leurs mélanges.

8. Composition de soins bucco-dentaires selon l'une quelconque des revendications précédentes comprenant en outre un humectant choisi parmi la glycérine, le sorbitol, le propylène glycol, le polyéthylène glycol, le xylitol et leurs mélanges.

9. Composition de soins bucco-dentaires selon l'une quelconque des revendications précédentes comprenant en outre une quantité modifiant la viscosité d'un ou plusieurs polymères choisis parmi les polyéthylèneglycols, les polysaccharides, les dérivés de la cellulose, la carboxyméthylcellulose, les gommes de polysaccharide, la gomme xanthane, la gomme carraghénane et leurs combinaisons.

10. Composition de soins bucco-dentaires selon l'une quelconque des revendications précédentes comprenant en outre un agent tampon, un aromatisant, un colorant, un parfum, un agent de blanchiment ou une combinaison de ceux-ci.

11. Composition de soins bucco-dentaires selon l'une quelconque des revendications précédentes comprenant en outre une quantité efficace d'un ou plusieurs agents antibactériens choisis dans le groupe constitué par l'éther diphénylique halogéné, le triclosan, les extraits de plantes, les huiles essentielles, l'extrait de romarin, l'extrait de thé, l'extrait de magnolia, le thymol, le menthol, l'eucalyptol, le géraniol, le carvacrol, le citral, l'hinokitol, le catéchol, le salicylate de méthyle, le gallate d'épi-gallocatéchine, l'épigallocatéchine, l'acide gallique, l'extrait de miswak, l'extrait d'argousier, les antiseptiques bisguanides, la chlorhexidine, l'alexidine ou l'octénidine, les composés d'ammonium quaternaire, le chlorure de cétylpyridinium (CPC), le chlorure de benzalkonium, le chlorure de tétradécylpyridinium (TPC), le chlorure de N-tétradécyl-4-éthylpyridinium (TDEPC), les antiseptiques phénoliques, l'hexétidine, l'octénidine, la sanguinarine, la povidone iodée, le delmopinol, le salifluor, les sels stanneux, les sels de cuivre, les sels de fer, la sanguinarine, la propolis et les agents oxygénants, le peroxyde d'hydrogène, le peroxyborate ou le peroxycarbonate de sodium tamponné, l'acide phtalique et ses sels, l'acide monoperthalique et ses sels et esters, le stéarate d'ascorbyle, l'oléoyl sarcosine, un sulfate d'alkyle, le dioctyl sulfosuccinate, le salicylanilide, le bromure de domiphène, le delmopinol, l'octapinol et autres dérivés de pipéridino, les préparations de nicine, les sels de chlorite et les mélanges de tout ce qui précède.

12. Composition de soins bucco-dentaires selon l'une quelconque des revendications précédentes, pour une utilisation dans la réduction ou l'inhibition de la formation de biofilm dans une cavité buccale, ou pour une utilisation dans la réduction ou l'inhibition de l'hypersensibilité dentinaire.

13. Composition de soins bucco-dentaires selon l'une quelconque des revendications précédentes, dans laquelle la composition comprend une source de fluorure, et dans laquelle la source de fluorure est un sel choisi parmi le fluorure stanneux, le fluorure de sodium, le fluorure de potassium, le fluorosilicate de sodium, le fluorosilicate d'ammonium, un fluorure d'amine, de préférence le N'-octadécyltriméthylènediamine-N,N,N'-tris(2-éthanol)-dihydrofluorure ; le fluorure d'ammonium, le fluorure de titane, l'hexafluorosulfate et leurs combinaisons.
